(19) Europäisches Patentamt / European Patent Office / Office européen des brevets

(11) EP 3 514 241 A1

(12) EUROPEAN PATENT APPLICATION

(43) Date of publication:
**24.07.2019 Bulletin 2019/30**

(51) Int Cl.:
***C12P 7/04*** *(2006.01)* ***C12P 7/24*** *(2006.01)*
***C12P 7/62*** *(2006.01)*

(21) Application number: **18152653.4**

(22) Date of filing: **19.01.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **ESIM Chemicals GmbH**
**4020 Linz (AT)**

(72) Inventors:
- **STROHMEIER, Gernot Alexander**
**8411 Hengsberg (AT)**
- **HÄUBL, Martin**
**4040 Linz (AT)**
- **SCHUECKER, Raffael**
**4020 Linz (AT)**
- **HOLUB, Bernhard**
**4072 Straßham (AT)**

(74) Representative: **Prüfer & Partner mbB**
**Patentanwälte · Rechtsanwälte**
**Sohnckestraße 12**
**81479 München (DE)**

Remarks:
Amended claims in accordance with Rule 137(2) EPC.

# (54) PROCESS FOR A MONOACYLATION OF DIOLS AND RELATED COMPOUNDS

(57) Process for a monoacylation of diols or related compounds by way of reaction with an acyl donor, wherein the initial concentration of the diol being at least 1 mol/L and wherein the reaction is carried out in the presence of an esterase.

EP 3 514 241 A1

## Description

The present application relates to a process for a monoacylation of organic diols and related compounds by way of reaction with an acyl donor in the presence of an esterase.

The enzymatic acylation of a diol leading to an excess of the monoacylated compound over the diacylated compound is described in the article by A. Hamberg et al., ChemCatChem Vol. 5, p. 743-747 (2013), the document WO 2011/033039 A1, and the article by V. Framis et al., Tetrahedron Letters Vol. 45, p. 5031-5033 (2004).

According to the literature, the enzymatic acylation is carried out by reacting the diol with an acyl donor in the presence of an esterase in a concentration regime characterized by a low concentration of the diol

It is an object of the present invention to provide an alternative, preferably improved process for a monoacylation of diols.

This object is achieved by the process according to claim 1. The features defined in the dependent claims as well as the features specified in the description below are refinements of the inventive process according to claim 1 and further contribute to the achievement of the surprising effects and additional unexpected advantages

The process according to the invention is a process for the production of a compound **III** represented by the following formula

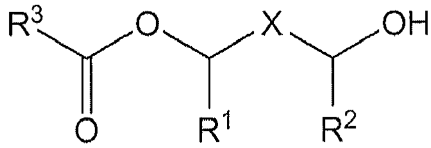

**(III)**

by a monoacylation of a compound **I** represented by the following formula

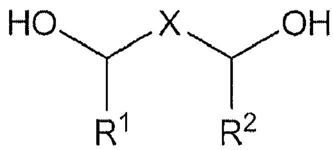

**(I)**

wherein compound **I** and a compound **II** represented by the following formula

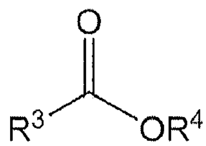

**(II)**

are reacted with one another in a reaction mixture at least initially comprising compounds **I** and **II.**

The initial concentration of compound **I** in the reaction mixture is at least 1 mol/L.

Compounds **I** and **II** are reacted with one another in the presence of at least one esterase.

Compound **II** acts as an acyl donor in the sense that the acyl groups $R^3$-(CO)- are transferred to the molecules of compound **I** in the course of the process.

-X- denotes a single bond or a structural moiety with n carbon atoms, wherein n is an integer number from 1 to 5.

-$R^1$ denotes hydrogen or a chemical group selected from the group consisting of unbranched or branched alkyl, unbranched or branched alkenyl, unbranched or branched alkynyl, unbranched or branched aralkyl, and aryl. The alkyl, the alkenyl, the alkynyl, the aralkyl, or the aryl in -$R^1$ may be either unsubstituted or substituted by at least one heteroatom and/or at least one heterogroup.

-$R^2$ denotes hydrogen or a chemical group selected from the group consisting of unbranched or branched alkyl, unbranched or branched alkenyl, unbranched or branched alkynyl, unbranched or branched aralkyl, and aryl. The alkyl, the alkenyl, the alkynyl, the aralkyl, or the aryl in -$R^2$ being may be either unsubstituted or substituted by at least one heteroatom and/or at least one heterogroup.

$-R^3$ denotes hydrogen or a chemical group selected from the group consisting of unbranched or branched alkyl, unbranched or branched alkenyl, unbranched or branched alkynyl, unbranched or branched aralkyl, and aryl. The alkyl, the alkenyl, the alkynyl, the aralkyl, or the aryl in $-R^3$ may be either unsubstituted or substituted by at least one heteroatom and/or at least one heterogroup.

$-R^4$ denotes hydrogen or a chemical group selected from the group consisting of unbranched or branched alkyl, unbranched or branched alkenyl, unbranched or branched alkynyl, unbranched or branched aralkyl, and aryl. The alkyl, the alkenyl, the alkynyl, the aralkyl, or the aryl in $-R^4$ may be either unsubstituted or substituted by at least one heteroatom and/or at least one heterogroup.

A heteroatom is understood as any atom different from carbon and hydrogen, such as for example fluorine, chlorine, bromine, and iodine. Accordingly, a heterogroup is understood as a group of covalently bonded atoms of which at least one is a heteroatom. Examples of heterogroups are hydroxyl groups, sulfhydryl groups, alkyloxy groups, primary, secondary, and tertiary amine groups, thioalkyloxy groups, organophosphourus groups and organoboron groups. The atoms of $-R^1$, $-R^2$, $-R^3$, and $-R^4$ bonded to the respective C- or O-atoms of the remaining molecule are either H-atoms or C-atoms, i.e. the atoms of $-R^1$, $-R^2$, $-R^3$, and $-R^4$ directly bonded to the core part of the molecules of compounds **I** and **II** are not heteroatoms. The core parts of the molecules of compounds **I** and **II** are the molecular backbones -CH(OH)-X-CH(OH)- and -(CO)-O-, respectively.

If one or more of the groups -X-, $-R^1$ to $-R^4$ are substituted by a heterogroup which can in principle undergo an acylation reaction, the esterase, compound **II**, and the reaction conditions are selected such that the heterogroup is not acylated to a considerable extent in order to avoid the formation of undesired by-products. Preferably, the groups -X-, $-R^1$ and $-R^2$ do not comprise heteroatoms or heterogroups such as hydroxyl groups and/or sulfhydryl groups which could undergo an acylation or detrimentally influence the reaction of compound **I** with compound **II**. If the hydroxyl groups shown in the formula of compound **I** are secondary hydoxyl groups, i.e. if $-R^1 = -R^2$ are not hydrogen atoms, it is particularly preferred that the groups -X-, $-R^1$ and $-R^2$ do not comprise primary hydroxyl groups.

Preferably, compound **I** is symmetrical, i.e. $-R^1 = -R^2$ in order to avoid the formation of $HO-CHR^1-X-CHR^2-O-(CO)-R^2$ being a constitutional isomer of compound **III**.

Surprisingly, it has been found that esterases can be stable and enzymatically active at high concentrations of compound **I,** viz. at a concentrations of 1 mol/L or even higher concentrations. Accordingly, it was found that despite the high concentrations of compound **I**, it is possible to obtain a high degree of conversion of compound **I** and a fast acylation reaction. This behaviour is unexpected mainly because compound **I** has hydroxyl (-OH) functional groups, i.e. alcohol groups, which presumably would be expected to lead to enzyme deactivation or even enzyme denaturation at such high concentrations. Usually, one would expect that enzymes are active in diluted aqueous systems only and furthermore that especially a high concentration of alcohol groups (and/or a high concentration of esters) would at least heavily affect or flaw the enzyme's activity or even irreversibly denaturize the enzyme. Such loss of activity or denaturation may for example be caused by the interference of alcohol molecules (including molecules of diols and polyols) with the enzyme's hydrate shell.

The unexpected possibility to use high alcoholic educt concentrations, however, makes the beneficial application of monoacylation feasible on an industrial scale; a high concentration of the educts may for instance allow for an effective utilization of the production facilities, a high production throughput, and an easier isolation of the product.

Esterases (EC 3.1) are hydrolase enzymes acting on ester bonds. Esterases may have the ability to catalyze an ester formation reaction. In the context of the invention, the subclass of triacylglycerol lipases (EC 3.1.1.3), which are hereinafter simply referred to as "lipases", and the subclass of cutinases (EC 3.1.1.74) have been found to be particularly useful esterases. Lipases and cutinases both belong to the enzyme class EC 3.1.1. The mentioned EC numbers follow the Enzyme Commission numbering system.

In the context of the invention, it is preferred that the at least one esterase is a lipase or cutinase, wherein more specifically the lipase or cutinase may be obtained from one of the organisms selected from the group consisting of *Candida antarctica, Rhizomucor miehei, Thermomyces languinosa, Candida rugosa, Pseudomonas cepacia, Pseudomonas fluorescens, Rhizopus oryzae, Mucor javanicus, Aspergillus niger, Rhizopus niveus, Alcaligenes* sp., *Fusarium solani pisi, Gandida cylindracea* sp. and *Bacillus subtilis.*

The *Candida antarctica* lipase B, which is one of the enzymes preferably used in the inventive process, is also referred to as "CaLB".

In the context of the invention, different esterase enzymes, e.g. a lipase and a cutinase, may be simultaneously used.

The initial concentration of a compound is understood as the concentration of this compound in the reaction mixture at the beginning of the reaction, i.e. for an extent of reaction $\xi = 0$. The initial concentration of compounds **I** and **II** in the reaction mixture are denoted as $c_I^0$ and $c_{II}^0$, respectively.

Preferably, $c_I^0$ is at least 1.5 mol/L, more preferably at least 2 mol/L, even more preferably at least 2.5 mol/L. In the context of the invention, it is also possible that $c_I^0$ adopts even higher values such as values $\geq 3$ mol/L.

Preferably, $c_{II}^0$ is at least 1 mol/L, more preferably at least 2 mol/L, even more preferably at least 4 mol/L, and

even more preferably at least 6 mol/L. In the context of the invention, it is also possible that $c_{II}^0$ adopts even higher values such as values ≥ 7.5 mol/L

Preferably, the ratio of the initial concentration of compounds **I** and **II**, $c_{II}^0/c_I^0$ is at least 1, more preferably at least 2, even more preferably at least 2.5, and most preferably at least 3.

If for example the following three conditions (i) to (iii) are fulfilled,

(i) 1,3-propanediol is used as compound **I** ($R^1 = R^2 = H$, $X = CH_2$),
(ii) methyl acetate is used as compound **II** ($R^3 = R^4 = CH_3$), and
(iii) $c_{II}^0/c_I^0 = 3$,

it can be estimated on the basis of the molar mass values and the density values of 1,3-propanediol ($M$ = 76 g/mol, $\rho$ = 1.06 g/mL) and methyl acetate ($M$ = 74 g/mol, $\rho$ = 0.93 g/mL) that the maximum possible value of $c_I^0$ is 1000/(76/1.06 + 3×74/0.93) ≈ 3 mol/L.

Compound III is formed by esterification of one of the hydroxyl groups of compound I (monoacylation). It is, however, possible that further esterification of compound **III** occurs (diacylation). This would lead to the formation of compound **IV** represented by the following formula

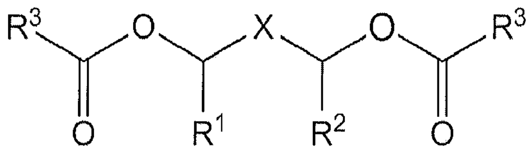

**(IV)**

In this formula, -X-, $-R^1$, $-R^2$, $-R^3$, and $-R^4$ are defined as above.
Compound **IV** is regarded as a by-product, i.e. a generally undesired incidental product.

Further surprisingly, it has been found that using the process according to the invention, the produced amount of compound **III** may be substantially larger than the produced amount of compound **IV** so that the acylation may essentially be regarded as monoacylation. In other words, the present invention allows the extent of diacyl formation to be substantially reduced, preferably to an inevitable minimum. Hence, the monoacylated product may be obtained with relatively low amounts of by-products.

The final concentration of a compound is understood as the concentration of this compound in the reaction mixture at the time when the reaction terminates or when the reaction is terminated. The final concentration of compounds **I**, **II**, **III**, and **IV** in the reaction mixture are denoted as $c_I^f$, $c_{II}^f$, $c_{III}^f$, and $c_{IV}^f$, respectively.

The ratio of $c_{III}^f$ and $c_{IV}^f$,

$$S = c_{III}^f / c_{IV}^f, \qquad (1)$$

can be regarded as a measure for the selectivity of the acylation reaction underlying the inventive process.

Preferably, the $S$ is at least 2, more preferably at least 4, even more preferably at least 10, and most preferably at least 20.

With a value of $S \geq 2$, it is avoided that large amounts of compound **IV** are formed. The formation of large amounts of compound **IV** would have adverse effect in terms of the chemical yield. Moreover, the formation of large amounts of compound **IV** would make the isolation of the desired product burdensome and costly.

In view of the high initial concentration of compound **I**, the achievement of a value of $S \geq 2$ is an unforeseeable result. It would rather be expected that the high concentration would counteract the enzyme's ability to selectively acylate only one of the OH-groups of compound **I**.

A high selectivity corresponding to $S \geq 2$ is particularly unexpected and highly beneficial if compound **II** is used in a hyperstoichiometric amount with respect to compound **I**, i.e. if $c_{II}^0/c_I^0 > 1$. The use of hyperstoichiometric amounts of compound **II** can also be referred to as using an "excess" of compound **II**. On the one hand, a hyperstoichiometric amount of compound **II** is expected to lead to a high degree of conversion of compound **I** due to the law of mass action; one has to bear in mind, that the alcohol $R^4$-OH formed as a by-product of the acylation reaction remains in the reaction mixture and that the reverse acylation reaction is favoured the higher the concentration of $R^4$-OH is. From this point of view, a hyperstoichiometric amount of compound **II** would be desirable. On the other hand, however, a high value of $c_{II}^0/c_I^0$ must be expected to be detrimental to the selectivity based on statistical considerations: Under the assumption that the acylation of compound **III** is neither favoured nor unfavoured compared to the acylation of compound I, for

instance $c_{II}^0/c_I^0$ = 1 corresponds to $S = c_{III}^f / c_{IV}^f = 2$ and with $c_{II}^0/c_I^0$ = 1.5 already a smaller amount of compound **III** would be formed than of compound **IV**.

If the acylation of a diol is carried out without the use of a catalytic enzyme, a statistical mixture of monoacylated and diacylated diol will result. In other words, the non-enzymatic acylation is not selective. Using the inventive process, selectivity becomes possible and can be achieved despite the high concentration of educt compound I and even at an excess of the acyl donor compound **II**. This means that the utilized enzymes have higher activity in respect to the first acylation of the diol as compared to their activity in respect of the second acylation. While such selectivity may be in line with expectations for low concentrations of the enzyme's substrate. However, it would have been expected that high concentrations of the substrate would lead to a reduced selectivity or even no selectivity at all as a sufficiently strong site-isolation effect can be expected only if the substrate concentration is low (generally expected to be due to the so-called dilution effect).

The dilution effect is expected to be only effective at low concentrations of the substrates. The reason for this behaviour may be that at high concentrations of the substrates, there is always a large amount of substrate bonded to the enzyme's active sites so that no site isolation or weak site isolation is observed. At low substrate concentrations, the saturation of the substrates' bonding to the active site is low so that the enzyme may more efficiently discriminate between compound **I** and compound **III**. Especially if the substrate molecules are small, it is expected that the monoacylated substrate, i.e. compound **III**, can also effectively bind to the active site and undergo a second acylation reaction. Therefore, a low selectivity is expected.

Accordingly, it is even more surprising that using the inventive process, selectivity may become achievable also when using an excess of the acyl donor. The excess of the acyl donor may have a bias towards high conversion of compound I. This may be true even if the concentration of compound I is high, viz. 1 mol/L or even higher. By using the inventive process, therefore, it may be possible to simultaneously apply two conditions (high excess and high concentration) that are usually considered as obstacles to a technically and economically useful reaction control and to benefit from using these conditions.

If compound **I** has a simple molecular structure, e.g. if compound **I** is a small symmetric diol such as 1,3-propanediol or 2,5-pentanediol, selectivity S is expected to be low because such simple molecular structure does not have structural motifs which usually provide for selectivity of an enzymatic reaction by way of selective bonding to the enzyme's active site. Surprisingly, high selectivity S has been found to be also possible for compounds **I** having simple molecular structures even for hyperstoichiometric amounts of compound **II**, if the process according to the invention is used.

Cutinase, which is one of the enzymes preferably used in the inventive process, is known to have a relatively large active site. It is therefore surprising that a high selectivity values such as $S \geq 2$ or even higher can be obtained even when using cutinase and a compound **I** with small molecules such as 1,3-prpanediol.

Preferably, the at least one esterase is immobilized on a carrier prior to reacting compound **I** and compound **II** with one another. The carrier together with the enzyme immobilized thereon is referred to as "enzyme preparation". An appropriate enzyme preparation may be prepared in advance. The carrier is preferably selected to be a solid carrier being essentially insoluble in the reaction mixture. This beneficially facilitates separation and optionally re-use of the esterase enzyme. The carrier may for instance be a porous absorber material. Absorber materials are commercially available from various manufacturers, e.g. under the brand names "LEWATIT® VP OC 1600" (Lanxess AG), "ACCUREL® MP" (Membrana/3M Company), IMMOBEAD IB-150A, carriers of the series LIFETECH ECR (Purolite Corp.) and comparable enzyme carriers.

Preferably, the amount of the at least one esterase being immobilized on the carrier is at least 10 mg, more preferably at least 25 mg, even more preferably at least 50 mg, and most preferably at least 100 mg per gram of the dry carrier. In the context of the invention, there may be even more esterase immobilized on the carrier, for instance 150 mg or even 200 mg per gram of the dry carrier.

Preferably, the amount of the at least one esterase being present in the reaction mixture is less than or equal to 1 g, more preferably less than or equal to 0.5 g, even more preferably less than or equal to 0.2 g, most preferably less than or equal to 0.1 g per mole of compound **I** being present in the reaction mixture before the compounds **I** and **II** are reacted with one another. If for instance the amount of the at least one esterase being present in the reaction mixture is selected to be 0.1 g per mole of compound **I** being present in the reaction mixture, if furthermore the amount of the at least one esterase being immobilized on the carrier is 100 mg per gram of the dry carrier, and if moreover 10 mol of compound **I** are used, 11 g of a composition consisting of the dry carrier and the esterase immobilized thereon are required.

The reaction mixture may comprise a solvent, preferably an organic solvent. Preferred organic solvents are methyl *tert*-butyl ether, cyclohexane, toluene, *o*-xylene, *m*-xylene, *p*-xylene, *n*-hexane, *n*-heptane, 2-butanone, *tert*-butanone, acetone as well as mixtures thereof.

Cyclohexane and/or toluene and/or other azeotrope-distillation solvents are especially convenient if water, which may be produced as a by-product of the reaction of compounds **I** and **II**, should be removed by a Dean-Stark trap or a similar apparatus; water is produced if compound **II** is a carboxylic acid such as acetic acid.

By removing water formed in the course of the reaction, the chemical equilibrium can be shifted in favour of the product due to Le Chatelier's principle.

A low water content may also be beneficial if compound **II** is different from a carboxylic acid (e.g. if compound **II** is an ester) in order to suppress the undesired hydrolysis of compound **II**. This may be achieved by using sufficiently dry chemicals. On the other hand, a certain water concentration may be maintained to ensure the enzyme's activity.

Acetone may act as a solubilizer ensuring that the reaction mixture is homogenous even if compounds **I** and **II** are per se immiscible at the high concentrations used. Therefore, acetone is particularly preferred among the aforementioned solvents.

The reaction mixture may also be free of any additional solvent. In this case, the reactants themselves act as the solvent. If, for example, methyl acetate or ethyl acetate is used as compound **II**, additional solvents may be unnecessary. This applies in particular for the use hyperstoichiometric amount of methyl acetate or ethyl acetate.

Preferably, the esterase, which is optionally immobilized on a carrier, is suspended in the reaction mixture. More preferably, the remaining reaction mixture is a solution.

Preferably, the compounds **I** and **II** are reacted with one another at a temperature of at least 10 °C, more preferably at least 25 °C, even more preferably at least 35 °C, most preferably at least 38 °C.

Preferably, the compounds I and **II** are reacted with one another at a temperature of 60 °C or less, more preferably 45 °C or less, most preferably 42 °C or less. In the context of the invention, temperatures up to 100 °C may be possible.

In order to remove water from the reaction mixture, it is possible to reduce the pressure acting upon the reaction mixture to a reduced pressure (i.e. a pressure below the atmospheric pressure) and distil the water off. Preferably, the reduced pressure is 500 mbar or less, more preferably 300 mbar or less, most preferably 175 mbar or less. In addition, it is preferred to use a reduced pressure of at least 50 mbar, preferably at least 100 mbar, more preferably at least 150 mbar.

The reaction period is understood as the amount of time compounds I and **II** are allowed to react with one another. After this period, the reaction is complete or terminated. Preferably, the reaction period is 3 days or less, more preferably 2 days or less, even more preferably 1 day or less, most preferably 18 hours or less. Furthermore, it is preferred to select a reaction period of at least 30 min, preferably at least 1 hour, more preferably at least 4 hours, most preferably at least 18 hours.

The inventive process can be carried out in as a batch process or fed-batch process. Alternatively, continuous processing is possible. For instance, continuous processing is preferred if the stability of the applied enzyme preparation is low under stirred conditions. In the case of a continuous process, the reaction period is the mean period time during which compounds **I** and **II** are allowed to react with one another in the presence of the esterase.

The ratio of the sum $c_{III}^f + c_{IV}^f$ and $c_I^0$,

$$C = ( c_{III}^f + c_{IV}^f ) / c_I^0 = ( c_{III}^f + c_{IV}^f ) / ( c_I^f + c_{III}^f + c_{IV}^f ) \quad (2)$$

can be regarded as a measure for the overall conversion, i.e. the yield of compounds **III** and **IV** (overall yield) obtained using the inventive process. Preferably, $C$ is at least 0.2, more preferably at least 0.5, even more preferably at least 0.75, most preferably at least 0.9.

The specific yield $D$ of compound **III**, i.e. the ratio of $c_{III}^f$ and $c_I^0$, can be calculated on the basis of $C$ and $S$:

$$D = c_{III}^f / c_I^0 = C \times S / (1 + S) \quad (3)$$

The groups -R$^1$ and -R$^2$ may be independently selected to be hydrogen or aliphatic chemical groups. Preferably, the aliphatic chemical groups are alkyl groups which may be either branched or unbranched. According to preferred embodiments of the invention, -R$^1$ and -R$^2$ are independently selected from the group consisting of hydrogen, methyl, ethyl, *n*-propyl, isopropyl, n-butyl, isobutyl, *sec*-butyl, and *tert*-butyl, wherein most preferably -R$^1$ and -R$^2$ both are hydrogen.

The group -X- may be selected from the group consisting of unbranched or branched alkylene, unbranched or branched alkenylene, unbranched or branched alkynylene, unbranched or branched aralkylene, and aromatic groups. The alkylene, the alkenylene, the alkynylene, the aralkylene, or the aromatic group of -X- can be either unsubstituted or substituted by at least one heteroatom and/or at least one heterogroup.

Preferably, -X- is represented by the following formula

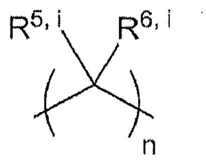

n is an integer number from 1 to 5 ($1 \leq n \leq 5$). i is an integer number adopting the values from 1 to n ($1 \leq i \leq n$). $-R^{5,i}$ and $-R^{6,i}$ are independently selected from the group consisting of hydrogen, unbranched or branched alkyl, unbranched or branched alkenyl, unbranched or branched alkynyl, unbranched or branched aralkyl, and aryl. The alkyl, the alkenyl, the alkynyl, the aralkyl, or the aryl in $R^{5,i}$ and $R^{6,i}$ may be either unsubstituted or substituted by at least one heteroatom and/or at least one heterogroup.

In this case, the carbon atoms bonded to the hydroxyl groups shown in the above formula of compound **I** are bridged by a carbon atom (if n = 1) or a chain having a carbon backbone (if n from 2 to 5).

According to particularly preferred embodiments, -X- is selected from the group consisting of $-CH_2-$, $-CH_2-CH_2-$, $-CH(CH_3)-$, $-CH_2-CH_2-CH_2-$, $-CH_2-CH(CH_3)-$, $-C(CH_3)_2-$, $-CH(CH_2-CH_3)-$, $-CH_2-CH_2-CH_2-CH_2-$, $-CH_2-CH_2-CH(CH_3)-$, $-CH_2-CH(CH_3)-CH_2$, $-CH(CH_3)-CH(CH_3)-$, $-CH_2-C(CH_3)_2-$, $-CH_2-CH(CH_2-CH_3)-$, $-CH(CH_2-CH_2-CH_3)-$, $-CH(CH(CH_3)_2)-$, and $-C(CH_2-CH_3)(CH_3)-$. Most preferably -X- is $-CH_2-$ (methylene).

Preferably, compound I is a diol, more preferably 1,3-propanediol.

If $-R^1 = -R^2 = H$ and $X = CH_2$, compound I is 1,3-propanediol.

Alternatively, -X- is represented by the formula -Y-A-Z-. -Y- and -Z- are independently selected from the group consisting of unbranched or branched alkylene, unbranched or branched alkenylene, unbranched branched alkynylene, branched or unbranched aralkylene, and aromatic groups. The alkylene, the alkenylene, the alkynylene, the aralkylene, or the aromatic group of -Y- and -Z- may be unsubstituted or substituted by at least one heteroatom and/or at least one heterogroup.

Preferably, -Y- and -Z- are independently selected from the group consisting of $-CH_2-$, $-CH_2-CH_2-$, $-CH(CH_3)-$, $-CH_2-CH_2-CH_2-$, $-CH_2-CH(CH_3)-$, $-C(CH_3)_2-$, $-CH(CH_2-CH_3)-$, $-CH_2-CH_2-CH_2-CH_2-$, $-CH_2-CH_2-CH(CH_3)-$, $-CH_2-CH(CH_3)-CH_2$, $-CH(CH_3)-CH(CH_3)-$, $-CH_2-C(CH_3)_2-$, $-CH_2-CH(CH_2-CH_3)-$, $-CH(CH_2-CH_2-CH_3)-$, $-CH(CH(CH_3)_2)-$ and $-C(CH_2-CH_3)(CH_3)$. More preferably, -Y- and -Z- are both $-CH_2-$.

-A- is a heteroatom or a heterogroup, preferably a heteroatom or a heterogroup selected from the group consisting of -O-, -S-, -NH- and $-NR^7-$. $-R^7$ is an alkyl group. Preferably, -A- is either -S- or $-NR^7-$.

Preferably, $-R^3$ and $-R^4$ are independently selected from the group consisting of hydrogen and aliphatic chemical groups, more preferably from the group consisting of hydrogen and unbranched or branched alkyl. Even more preferably, $-R^3$ is selected from the group consisting of hydrogen, methyl, ethyl, *n*-propyl, isopropyl, *n*-butyl, isobutyl, *sec*-butyl, *tert*-butyl and/or $-R^4$ is selected from the group consisting of hydrogen, methyl, ethyl, *n*-propyl, isopropyl, vinyl, propenyl, allyl, isopropenyl.

According to preferred embodiments of the invention, compound **II** is selected from the group consisting of methyl acetate, ethyl acetate, *n*-propylacetate, isopropyl acetate, vinyl acetate, propenyl acetate, isopropenyl acetate, acetic acid, acetate with an inorganic or organic cation, acetic anhydride.

Most preferably, compound **II** is either methyl acrylate or ethyl acrylate.

In the context of the invention, compound **II** may be a dicarbonyl compound represented by the formula

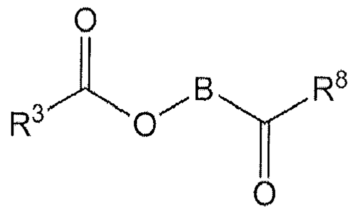

$-R^3$ being defined as above. $-R^8$ is selected from the group consisting of hydrogen, unbranched or branched alkyl, unbranched or branched alkenyl, unbranched or branched alkynyl, branched or unbranched aralkyl, and aryl. The alkyl, the alkenyl, the alkynyl, the aralkyl, or the aryl in $-R^8$ may be either unsubstituted or substituted by at least one heteroatom and/or at least one heterogroup. -B- may be either a single bond or a structural moiety selected from the group consisting of unbranched or branched alkylene, unbranched or branched alkenylene, unbranched or branched alkynylene, branched or unbranched aralkylene, and aromatic groups. The alkylene, the alkenylene, the alkynylene, the aralkylene, or the aromatic group of B may be either unsubstituted or substituted by at least one heteroatom and/or at least one heterogroup.
wherein preferably the compound **II** is an anhydride,
wherein more preferably the compound **II** is acetic anhydride.

## Examples

It is the purpose of the subsequently described examples to further illustrate the present invention. The examples do not restrict the scope of the invention. In the context of the entire disclosure, it is evident that modifications and amendments can be implemented.

### Materials:

The chemicals used in the examples were obtained from Sigma-Aldrich Corp. if not otherwise mentioned. 1,3-propanediol (Sigma-Aldrich, no. P50404-100g; lot: STBB7627V) was used after vacuum distillation at 5 mbar (bp 93°C) using a 20 cm Vigreux column.

Novozym 435 is the tradename of a commercially available enzyme preparation comprising CaLB enzyme (Candida antarctica lipase B) immobilized on Lewatit® VP OC 1600. For the use in the examples, Novozym 435 was purchased from Sigma-Aldrich Corp. and used as received.

In the examples, cutinase was used in the form of a commercially available enzyme preparation comprising the enzyme obtained from *Fusarium solani pisi* immobilized on IMMOBEAD IB-150A. The enzyme preparation was purchased under the tradename "Cutinase" (product no. IMML51-T2-150) from ChiralVision B.V., Leiden, The Netherlands and used as received.

### GC Analyses:

Gaschromatographic (GC) analyses were performed in order to measure the concentrations of 1,3-propanediol (compound **I**), 1,3-propanediol monoacetate (compound **III**), and 1,3-propanediol diacetate (compound **IV**) in the reaction mixtures. GC analyses were performed on an Agilent 6890N Network GC System equipped with a 7683 Series Injector and a flame ionization detector using a DB-1701 capillary column (Agilent 1220732; 30 m × 0.25 mm, 0.25 μm film thickness). The following settings were used: inlet temperature: 250°C; detector temperature: 250°C; 1.0 mL/min flow rate of $N_2$ carrier gas.

The following temperature program was applied: The temperature of 90°C was held for 1 min, then the temperature was increased with a rate of 20°C/min to 150°C, then the temperature was increased with a rate of 50°C/min to 220°C, and then the temperature was held for 0.6 min.

Using the described GC method, the following retention times were measured: 1,3-propanediol: 2.58 min,
1,3-propanediol monoacetate: 3.17 min,
1,3-propanediol diacetate: 3.87 min.

The peak areas of the GC-FID chromatograms were determined by integration with the Chemstation software package (Agilent) and used to calculate the concentrations. Different responses of the compounds were compensated based on their number of carbon atoms, assuming a linear dependency of the FID signal on the carbon fraction.

On the basis of the measured final concentrations of compounds **III** and **IV** ($c_{III}^f$ and $c_{IV}^f$) and on the basis of the initial concentrations of compounds **I** and **II** ($c_I^0$ and $c_{II}^0$), the values of $c_{II}^0$ / $c_I^0$, *S* and C were calculated.

### Enzymatic acylation reaction:

To 10 mg of the enzyme preparation (Novozym 435 or Cutinase as described above), 500 μL of a solution of compound **I**, compound **II** and a solvent was added. The respective amounts are given in Tables 1, 3, and 5. The reactions were conducted in sealed 1.5 mL snap ring glass vials (purchased from La-Pha-Pack GmbH, Germany) while keeping them in a thermostatted environment at a shaking speed of 130 rpm at 40°C for the respective reaction period by using an incubation shaker.

In order to perform GC measurements, samples having a volume of 10 μL were taken from the reaction mixtures and diluted 1:50 with 490 μL dichloromethane prior to GC analysis.

In the case of Examples 1 and 2, homogenous reaction mixtures could be obtained by using methyl *tert*-butyl ether (MTBE). In the case of Example 3, acetone has been used as solubilizer.

### Example 1 (Comparative)

Four different solutions of 1,3-propanediol (compound **I**), methyl acetate, ethyl acetate (compound **II**) and MTBE were used in Example 1. A volume of 500 μL of each solution was added to 10 mg of the enzyme preparation (Novozym 435 or Cutinase as described above) in order to perform the enzymatic acylation as described above. The volumes of 1,3-propanediol, methyl acetate of ethyl acetate, and MTBE are given in Table 1. These volumes determine the initial concentrations $c_I^0$ and $c_{II}^0$ which can be calculated based on these volumes and the values of the molar mass *M* and

density $\rho$ of 1,3-propanediol ($M$ = 76.09 g/mol, $\rho$ = 1.053 g/mL), methyl acetate ($M$ = 74.08 g/mol, $\rho$ = 0.932 g/mL), and ethyl acetate ($M$ = 88.11 g/mol, $\rho$ = 0.902 g/mL).

According to Example 1, the initial concentration of compound I is 0.05 mol/L.

Table 1

| Solution no. | Compound **II** | $c_{II}^0$ / $C_I^0$ | Volume of 1,3-propanediol [μL] | Volume of com-pound **II** [μL] | Volume of MTBE [μL] |
|---|---|---|---|---|---|
| 1 | methyl acetate | 1 | 1.81 | 1.99 | 496.2 |
| 2 | methyl acetate | 4 | 1.81 | 7.95 | 490.2 |
| 3 | ethyl acetate | 1 | 1.81 | 2.44 | 495.8 |
| 4 | ethyl acetate | 4 | 1.81 | 9.77 | 488.4 |

The results obtained for C and S after a reaction period of 18 hours are given in Table 2.

Table 2

| Enzyme | Compound **II** | $c_{II}^0/c_I^0$ | Reaction period | $C$ [%] | $S$ |
|---|---|---|---|---|---|
| Novozym 435 | methyl acetate | 1 | 18 h | 49% | 10.4 |
| | | 4 | 18 h | 80% | 2.8 |
| | ethyl acetate | 1 | 18 h | 52% | 8.0 |
| | | 4 | 18 h | 87% | 2.1 |
| Cutinase | methyl acetate | 1 | 18 h | 40% | 11.1 |
| | | 4 | 18 h | 77% | 3.7 |
| | ethyl acetate | 1 | 18 h | 48% | 8.9 |
| | | 4 | 18 h | 83% | 2.9 |

If no excess of compound **II** is used, i.e. if $c_{II}^0$ / $c_I^0$ = 1, relatively incomplete conversion is observed after a reaction period of 18 hours: The respective values of C lie between 40% and 52%. However, the reaction is selective (S lies between 8.0 and 11.1). This behaviour is observed irrespective of whether methyl acetate or ethyl acetate is used as acyl donor (compound **II**).

If a four-fold excess of compound **II** is used, i.e. if $c_{II}^0$ / $c_I^0$ = 4, the conversion observed after a reaction period of 18 hours is significantly increased. The respective values of C lie between 77% and 87%. The selectivity, however is decreased in line with the general expectations (see above). For $S$, values as low as 2.1 to 3.7 have been observed. This behaviour is observed irrespective of whether methyl acetate or ethyl acetate is used as acyl donor (compound **II**).

The comparison of the results for $c_{II}^0$ / $c_I^0$ = 1 and $c_{II}^0$ / $c_I^0$ = 4 demonstrates that the relative amount of compound **II** has opposite effects in view of conversion, i.e. yield, on the one hand and selectivity on the other hand counteract.

**Example 2 (Comparative)**

Two different solutions of 1,3-propanediol (compound I), methyl acetate, ethyl acetate (compound **II**) and MTBE were used in Example 2. A volume of 500 μL of each solution was added to 10 mg of the enzyme preparation (Novozym 435 or Cutinase as described above) in order to perform the enzymatic acylation as described above. The volumes of 1,3-propanediol, methyl acetate of ethyl acetate, and MTBE are given in Table 3. These volumes determine the initial concentrations $c_I^0$ and $c_{II}^0$ which can be calculated based on these volumes and the values of the molar mass $M$ and density $\rho$ of 1,3-propanediol ($M$ = 76.09 g/mol, $\rho$ = 1.053 g/mL), methyl acetate ($M$ = 74.08 g/mol, $\rho$ = 0.932 g/mL), and ethyl acetate ($M$ = 88.11 g/mol, $\rho$ = 0.902 g/mL).

According to Example 2, the initial concentration of compound I is 0.5 mol/L.

Table 3

| Solution no. | Compound **II** | $c_{II}^0$ / $c_I^0$ | Volume of 1,3-propanediol [μL] | Volume of com-pound **II** [μL] | Volume of MTBE [μL] |
|---|---|---|---|---|---|
| 5 | methyl acetate | 20 | 9.03 | 198.71 | 292.3 |
| 6 | ethyl acetate | 20 | 9.03 | 244.21 | 246.8 |

The results obtained for *C* and *S* after a reaction time of 1 hour are given in Table 4.

Table 4

| Enzyme | Compound **II** | $c_{II}^0/c_I^0$ | Reaction period | C [%] | *S* |
|---|---|---|---|---|---|
| Novozym 435 | methyl acetate | 20 | 1 h | 96% | 0.6 |
| | ethyl acetate | 20 | 1 h | 85% | 0.4 |
| Cutinase | methyl acetate | 20 | 1 h | 86% | 1.1 |
| | ethyl acetate | 20 | 1 h | 69% | 0.9 |

The excess of compound **II** used in Example 2 is much higher ($c_{II}^0$ / $c_I^0$ = 20) compared to Example 1 ($c_{II}^0$ / $c_I^0$ = 1 and $c_{II}^0$ / $c_I^0$ = 4). In line with the results obtained for Example 1, the increase of the relative amount of compound **II** has a strong impact on the selectivity. S adopts values below or slightly above 1, i.e. the produced amount of the by-product (compound **IV**) is smaller or almost equal to the produced amount of the desired product (compound **III**). For industrial applications, this is considered inappropriate and disadvantageous.

The conversion may be increased compared to Example 1 with $c_{II}^0$ / $c_I^0$ = 4; only the use of ethyl acetate together with cutinase as esterase has led to a reduction of *C*. However, one has to bear in mind, that the initial concentration of compound **I,** $c_I^0$, is ten times higher in the case of Example 2 so that a *C* value of 69% still corresponds to a much higher amount of compound **III** than a *C* value of 83% in Example 1.

**Example 3 (according to the present invention)**

Two different solutions of 1,3-propanediol (compound **I**), methyl acetate, ethyl acetate (compound **II**) and acetone were used in Example 3. A volume of 500 μL of each solution was added to 5 mg of the enzyme preparation (Novozym 435 or Cutinase as described above) in order to perform the enzymatic acylation as described above.

The volumes of 1,3-propanediol, methyl acetate of ethyl acetate, and acetone are given in Table 5. These volumes determine the initial concentrations $c_I^0$ and $c_{II}^0$ which can be calculated based on these volumes and the values of the molar mass *M* and density $\rho$ of 1,3-propanediol (*M* = 76.09 g/mol, $\rho$ = 1.053 g/mL), methyl acetate (*M* = 74.08 g/mol, $\rho$ = 0.932 g/mL), and ethyl acetate (*M* = 88.11 g/mol, $\rho$ = 0.902 g/mL).

According to example 3, the initial concentration of compound I is 2.5 mol/L, which is within the concentration range according to the invention.

Table 5

| Solution no. | Compound **II** | $c_{II}^0$ / $c_I^0$ | Volume of 1,3-propanediol [μL] | Volume of com-pound **II** [μL] | Volume of acetone [μL] |
|---|---|---|---|---|---|
| 7 | methyl acetate | 1.0 | 90.33 | 99.36 | 310.32 |
| 8 | methyl acetate | 1.5 | 90.33 | 149.03 | 260.64 |
| 9 | methyl acetate | 2.0 | 90.33 | 198.71 | 210.96 |
| 10 | methyl acetate | 3.0 | 90.33 | 298.07 | 111.61 |
| 11 | ethyl acetate | 1.0 | 90.33 | 122.10 | 287.57 |
| 12 | ethyl acetate | 1.5 | 90.33 | 183.16 | 226.52 |

(continued)

| Solution no. | Compound **II** | $c_{II}^0$ / $c_I^0$ | Volume of 1,3-propanediol [μL] | Volume of com-pound **II** [μL] | Volume of acetone [μL] |
|---|---|---|---|---|---|
| 13 | ethyl acetate | 2.0 | 90.33 | 244.21 | 165.47 |
| 14 | ethyl acetate | 3.0 | 90.33 | 366.31 | 43.36 |

The results obtained for *C* and *S* after a reaction time of 18, 42, or 120 hours are given in Table 6. In order to put emphasis on the fact that the desired compound **III** could be produced using the inventive process, values for *D* calculated according to Equation (3) are also stated in Table 6.

Table 6

| Enzyme | Compound **II** | $c_{II}^0/c_I^0$ | Reaction period | C [%] | D[%] | *S* |
|---|---|---|---|---|---|---|
| | | 1 | 18 h | 51% | 45% | 7.3 |
| | | 1.5 | 18 h | 47% | 41% | 7.0 |
| | | | 42 h | 54% | 46% | 5.7 |
| | methyl acetate | 2 | 18 h | 53% | 45% | 5.7 |
| | | | 42 h | 62% | 51% | 4.5 |
| | | 3 | 18 h | 57% | 48% | 5.0 |
| 435 | | | 42 h | 68% | 53% | 3.7 |
| Novozym | | 1 | 18 h | 55% | 47% | 5.7 |
| | | 1.5 | 18 h | 50% | 43% | 5.8 |
| | | | 42 h | 62% | 51% | 4.4 |
| | ethyl acetate | 2 | 18 h | 58% | 48% | 4.8 |
| | | | 42 h | 70% | 54% | 3.4 |
| | | 3 | 18 h | 76% | 55% | 2.7 |
| | | | 42 h | 80% | 55% | 2.2 |
| | | 1 | 18 h | 38% | 35% | 13 |
| | | 1.5 | 18 h | 36% | 34% | 16 |
| | | | 42 h | 48% | 44% | 10.3 |
| | | | 120 h | 52% | 46% | 7.2 |
| | methyl acetate | 2 | 18 h | 32% | 31% | 19 |
| | | | 42 h | 44% | 41% | 12.1 |
| | | | 120 h | 54% | 48% | 7.8 |
| | | 3 | 18 h | 44% | 41% | 13.3 |
| | | | 42 h | 57% | 51% | 8.2 |
| Cutinase | | | 120 h | 66% | 55% | 5.2 |

(continued)

| Enzyme | Compound **II** | $c_{II}^0/c_I^0$ | Reaction period | C [%] | D[%] | *S* |
|---|---|---|---|---|---|---|
| | ethyl acetate | 1 | 18 h | 42% | 39% | 12 |
| | | 1.5 | 18 h | 37% | 35% | 17 |
| | | | 42 h | 52% | 47% | 8.9 |
| | | | 120 h | 62% | 52% | 5.3 |
| | | 2 | 18 h | 43% | 40% | 12.7 |
| | | | 42 h | 60% | 52% | 6.8 |
| | | | 120 h | 69% | 55% | 4.0 |
| | | 3 | 18 h | 46% | 42% | 13.2 |
| | | | 42 h | 64% | 56% | 6.5 |
| | | | 120 h | 76% | 58% | 3.4 |

The results in table 6 show that at least satisfactory, yet with longer reaction periods highly beneficial *C* values can be achieved even with a high initial concentration of compound **I** and even if no excess of compound **II** is used, i.e. if $c_{II}^0$ / $c_I^0$ = 1. In view of the high initial concentration of compound **I**, *C* values of 32% or higher and especially *D* values of 31% or higher correspond to a reasonable and significantly high throughput on an industrial scale. By increasing $c_{II}^0$ / $c_I^0$ and/or the reaction period, *C* can be further increased.

It is a surprising and unforeseeable result that the enzymes maintain their activity in such concentration regimes at all. Even more surprising is the observed behaviour with respect to selectivity: Even for higher values of $c_{II}^0$ / $c_I^0$, *S* $\geq$ 2.2 is obtained.

Hence, a highly beneficial balance between conversion rate (yield) and selectivity S could be achieved.

Example 3 demonstrates, that using the inventive process, selectivity becomes achievable with high initial diol concentrations and even when using an excess of the acyl donor. It further demonstrates that it is sufficient to select $c_{II}^0$ / $c_I^0 \leq 3$ in order to simultaneously obtain high conversion and good selectivity and thereby balancing relevant counter-acting effects.

## Claims

1. A process for the production of compound III represented by the following formula

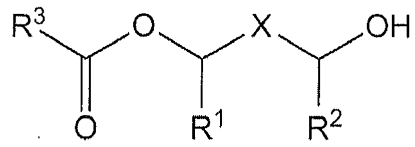

**(III)**

by a monoacylation of a compound **I** represented by the following formula

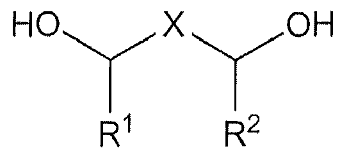

**(I)**

wherein compound **I** and a compound **II** represented by the following formula

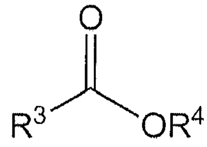

**(II)**

are reacted with one another in a reaction mixture at least initially comprising compounds **I** and **II**;
wherein the initial concentration of compound **I** in the reaction mixture is denoted as $c_I^0$;
wherein $c_I^0$ is at least 1 mol/L;
wherein compounds **I** and **II** are reacted with one another in the presence of at least one esterase;
wherein -X- denotes a single bond or a structural moiety with n carbon atoms, wherein n is an integer number from 1 to 5;
wherein -$R^1$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^1$ being unsubstituted or substituted by at least one heteroatom and/or at least one heterogroup;
wherein -$R^2$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^2$ being unsubstituted or substituted by at least one heteroatom and/or at least one heterogroup;
wherein -$R^3$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^3$ being unsubstituted or substituted by at least one heteroatom and/or at least one heterogroup;
wherein -$R^4$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^4$ being unsubstituted or substituted by at least one heteroatom and/or at least one heterogroup.

**2.** The process as claimed in claim 1,
wherein $c_I^0$ is at least 1.5 mol/L, preferably at least 2 mol/L, more preferably at least 2.5 mol/L, and/or
wherein the initial concentration of compound **II** in the reaction mixture being denoted as $c_{II}^0$ is at least 1 mol/L, preferably at least 2 mol/L, more preferably at least 4 mol/L, most preferably at least 6 mol/L.

**3.** The process as claimed in any of the preceding claims,
wherein the at least one esterase belongs to the enzyme class EC 3.1.1,
wherein preferably the at least one esterase is a lipase or cutinase,
wherein more preferably the at least one esterase is a lipase or cutinase from one of the organisms selected from the group consisting of *Candida antarctica, Rhizomucor miehei, Thermomyces languinosa, Candida rugosa, Pseudomonas cepacia, Pseudomonas fluorescens, Rhizopus oryzae, Mucor javanicus, Aspergillus niger, Rhizopus niveus, Alcaligenes* sp., *Fusarium solani pisi, Candida cylindracea* sp. and *Bacillus subtilis,*
wherein even more preferably the at least one esterase is a cutinase or a *Candida antarctica* lipase B,

wherein most preferably the at least one esterase is a *Candida antarctica* lipase B.

**4.** The process as claimed in one of the preceding claims,
wherein the amount of the at least one esterase being present in the reaction mixture is less than or equal to 1 g, preferably less than or equal to 0.5 g, more preferably less than or equal to 0.2 g, most preferably less than or equal to 0.1 g per mole of compound **I** being present in the reaction mixture before the compounds I and **II** are reacted with one another.

**5.** The process as claimed in one of the preceding claims,
wherein the reaction mixture comprises a solvent,
wherein preferably the solvent is an organic solvent,
wherein more preferably the solvent is selected from the group consisting of methyl *tert*-butyl ether, cyclohexane, toluene and acetone,
wherein most preferably the solvent is acetone.

**6.** The process as claimed in one of claims 1 to 4, wherein the reaction mixture does essentially not contain any solvent.

**7.** The process as claimed in one of the preceding claims, wherein the esterase, optionally immobilized, is suspended in the reaction mixture, wherein preferably the remaining reaction mixture is a solution.

**8.** The process as claimed in one of the preceding claims, wherein the compounds **I** and **II** are reacted with one another at a temperature of at least 10 °C, preferably at least 25 °C, more preferably at least 35 °C, most preferably at least 38 °C, and/or wherein the compounds **I** and **II** are reacted with one another at a temperature of 100 °C or less, preferably 60 °C or less, more preferably 45 °C or less, most preferably 42 °C or less.

**9.** The process as claimed in one of the preceding claims, wherein, in the reaction mixture after reacting compounds **I** and **II** are reacted with one another, the compound **III** has a final concentration denoted by $c_{III}{}^{f}$ and compound **IV** represented by the following formula

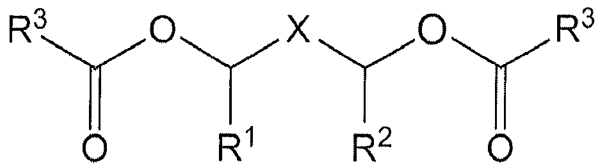

**(IV)**

being formed as a by-product when reacting compounds **I** and **II** is denoted by $c_{IV}{}^{f}$, wherein -X-, -$R^1$, -$R^2$, -$R^3$, and -$R^4$ are defined as in claim 1, wherein the ratio of $c_{III}{}^{f}$ and $c_{IV}{}^{f}$, $c_{III}{}^{f}/ c_{IV}{}^{f}$, is at least 2, preferably at least 4, more preferably at least 10, most preferably at least 20.

**10.** The process as claimed in one of the preceding claims, wherein, in the reaction mixture after reacting compounds **I** and **II** are reacted with one another, the compound **III** has a final concentration denoted by $c_{III}{}^{f}$, wherein the ratio of $c_{III}{}^{f}$ and $c_I{}^0$, $c_{III}{}^{f}/ c_I{}^0$, is at least 0.2, preferably at least 0.5, more preferably at least 0.75, most preferably at least 0.9.

**11.** The process as claimed in any of the preceding claims, wherein -$R^1$ and -$R^2$ are independently selected from the group consisting of hydrogen and aliphatic chemical groups; wherein preferably -$R^1$ and -$R^2$ are independently selected from the group consisting of hydrogen and unbranched or branched alkyl; wherein more preferably -$R^1$ and -$R^2$ are independently selected from the group consisting of hydrogen, methyl, ethyl, n-propyl, isopropyl, n-butyl, isobutyl, sec-butyl, and *tert*-butyl; wherein most preferably -$R^1$ and -$R^2$ both are hydrogen.

**12.** The process as claimed in one of the preceding claims, wherein -X- is selected from the group consisting of unbranched or branched alkylene, unbranched or branched alkenylene, unbranched or branched alkynylene, unbranched or branched aralkylene, and aromatic groups; the alkylene, the alkenylene, the alkynylene, the aralkylene, or the aromatic group of -X- being unsubstituted or substituted by at least one heteroatom and/or at least one heterogroup; wherein preferably -X- is represented by the following formula

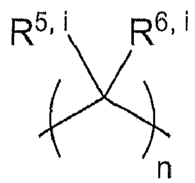

with n being an integer number from 1 to 5,
with i being an integer number adopting the values from 1 to n,
with $-R^{5,i}$ and $-R^{6,i}$ being independently selected from the group consisting of hydrogen, unbranched or branched alkyl, unbranched or branched alkenyl, unbranched or branched alkynyl, unbranched or branched aralkyl, and aryl, wherein the alkyl, the alkenyl, the alkynyl, the aralkyl, or the aryl in $R^{5,i}$ and $R^{6,i}$ is unsubstituted or substituted by at least one heteroatom and/or at least one heterogroup;
wherein more preferably -X- is selected from the group consisting of $-CH_2-$, $-CH_2-CH_2-$, $-CH(CH_3)-$, $-CH_2-CH_2-CH_2-$, $-CH_2-CH(CH_3)-$, $-C(CH_3)_2-$, $-CH(CH_2-CH_3)-$, $-CH_2-CH_2-CH_2-CH_2-$, $-CH_2-CH_2-CH(CH_3)-$, $-CH_2-CH(CH_3)-CH_2$, $-CH(CH_3)-CH(CH_3)-$, $-CH_2-C(CH_3)_2-$, $-CH_2-CH(CH_2-CH_3)-$, $-CH(CH_2-CH_2-CH_3)-$, $-CH(CH(CH_3)_2)-$ and $-C(CH_2-CH_3)(CH_3)-$,
wherein most preferably -X- is $-CH_2-$.

**13.** The process as claimed in one of claims 1 to 11,
wherein -X- is represented by the formula -Y-A-Z-
with -Y- and -Z- being independently selected from the group consisting of unbranched or branched alkylene, unbranched or branched alkenylene, unbranched branched alkynylene, branched or unbranched aralkylene, and aromatic groups; the alkylene, the alkenylene, the alkynylene, the aralkylene, or the aromatic group of -Y- and -Z- being unsubstituted or substituted by at least one heteroatom and/or at least one heterogroup; preferably, -Y- and -Z- being independently selected from the group consisting of
$-CH_2-$, $-CH_2-CH_2-$, $-CH(CH_3)-$, $-CH_2-CH_2-CH_2-$, $-CH_2-CH(CH_3)-$, $-C(CH_3)_2-$, $-CH(CH_2-CH_3)-$, $-CH_2-CH_2-CH_2-CH_2-$, $-CH_2-CH_2-CH(CH_3)-$, $-CH_2-CH(CH_3)-CH_2$, $-CH(CH_3)-CH(CH_3)-$, $-CH_2-C(CH_3)_2-$, $-CH_2-CH(CH_2-CH_3)-$, $-CH(CH_2-CH_2-CH_3)-$, $-CH(CH(CH_3)_2)-$ and $-C(CH_2-CH_3)(CH_3)$; and more preferably -Y- and -Z- both being $-CH_2-$; and
with -A- being a heteroatom or a heterogroup, preferably a heteroatom or a heterogroup selected from the group consisting of -O-, -S-, -NH- and $-NR^7-$ with $-R^7$ being an alkyl group.

**14.** The process as claimed in any of the preceding claims,
wherein $-R^3$ and $-R^4$ are independently selected from the group consisting of hydrogen and aliphatic chemical groups;
wherein preferably $-R^3$ and $-R^4$ are independently selected from the group consisting of hydrogen and unbranched or branched alkyl;
wherein more preferably $-R^3$ is selected from the group consisting of hydrogen, methyl, ethyl, *n*-propyl, isopropyl, *n*-butyl, isobutyl, *sec*-butyl, *tert-butyl* and/or $-R^4$ is selected from the group consisting of hydrogen, methyl, ethyl, n-propyl, isopropyl, vinyl, propenyl, allyl, isoprenyl;
wherein most preferably $-R^3$ is methyl and/or $-R^4$ is methyl or ethyl.

**15.** The process as claimed in any of the preceding claims,
wherein the compound **II** is a dicarbonyl compound represented by the formula

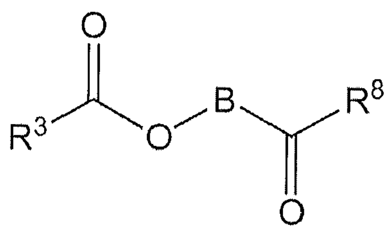

with $-R^3$ being defined as in claim 1,
with $-R^8$ being selected from the group consisting of hydrogen, unbranched or branched alkyl, unbranched or branched alkenyl, unbranched or branched alkynyl, branched or unbranched aralkyl, and aryl; the alkyl, the alkenyl, the alkynyl, the aralkyl, or the aryl in $-R^8$ being unsubstituted or substituted by at least one heteroatom and/or at least one heterogroup; and
with -B- being a single bond or a structural moiety selected from the group consisting of unbranched or branched alkylene, unbranched or branched alkenylene, unbranched or branched alkynylene, branched or unbranched aralkylene, and aromatic groups; the alkylene, the alkenylene, the alkynylene, the aralkylene, or the aromatic group of -B- being unsubstituted or substituted by at least one heteroatom and/or at least one heterogroup;
wherein preferably the compound **II** is an anhydride,
wherein more preferably the compound **II** is acetic anhydride.

**Amended claims in accordance with Rule 137(2) EPC.**

1. A process for the production of compound **III** represented by the following formula

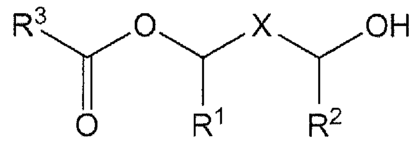

**(III)**

by a monoacylation of a compound **I** represented by the following formula

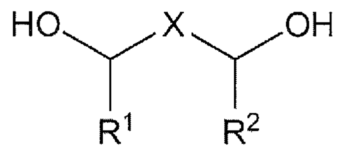

**(I)**

wherein compound **I** and a compound **II** represented by the following formula

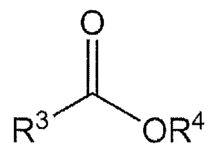

**(II)**

are reacted with one another in a reaction mixture at least initially comprising compounds **I** and **II;**
wherein the initial concentration of compound **I** in the reaction mixture is denoted as $c_I^0$;
wherein $c_I^0$ is at least 1 mol/L;
wherein compounds **I** and **II** are reacted with one another in the presence of at least one esterase;
wherein -X- denotes a single bond or a structural moiety with n carbon atoms, wherein n is an integer number from 1 to 5;
wherein -$R^1$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^1$ being unsubstituted or substituted by at least one heteroatom and/or at least one heterogroup;
wherein -$R^2$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^2$ being unsubstituted or substituted by at least one heteroatom and/or at least one heterogroup;
wherein -$R^3$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl, or the aryl in -$R^3$ being unsubstituted or substituted by at least one heteroatom and/or at least one heterogroup;
wherein -$R^4$ denotes hydrogen or a chemical group selected from the group consisting of unbranched or branched alkyl, unbranched or branched alkenyl, unbranched or branched alkynyl, unbranched or branched aralkyl, and aryl; the alkyl, the alkenyl, the alkynyl, the aralkyl ,or the aryl in -$R^4$ being unsubstituted or substituted by at least one heteroatom and/or at least one heterogroup.

2. The process as claimed in claim 1,
wherein $c_I^0$ is at least 1.5 mol/L, preferably at least 2 mol/L, more preferably at least 2.5 mol/L, and/or
wherein the initial concentration of compound **II** in the reaction mixture being denoted as $c_{II}^0$ is at least 1 mol/L, preferably at least 2 mol/L, more preferably at least 4 mol/L, most preferably at least 6 mol/L.

**3.** The process as claimed in any of the preceding claims, wherein the at least one esterase belongs to the enzyme class EC 3.1.1, wherein preferably the at least one esterase is a lipase or cutinase, wherein more preferably the at least one esterase is a lipase or cutinase from one of the organisms selected from the group consisting of *Candida antarctica, Rhizomucor miehei, Thermomyces languinosa, Candida rugosa, Pseudomonas cepacia, Pseudomonas fluorescens, Rhizopus oryzae, Mucor javanicus, Aspergillus niger, Rhizopus niveus, Alcaligenes* sp., *Fusarium solani pisi, Candida cylindracea* sp. and *Bacillus subtilis,* wherein even more preferably the at least one esterase is a cutinase or a *Candida antarctica* lipase B, wherein most preferably the at least one esterase is a *Candida antarctica* lipase B.

**4.** The process as claimed in one of the preceding claims, wherein the amount of the at least one esterase being present in the reaction mixture is less than or equal to 1 g, preferably less than or equal to 0.5 g, more preferably less than or equal to 0.2 g, most preferably less than or equal to 0.1 g per mole of compound **I** being present in the reaction mixture before the compounds **I** and **II** are reacted with one another.

**5.** The process as claimed in one of the preceding claims, wherein the reaction mixture comprises a solvent, wherein preferably the solvent is an organic solvent, wherein more preferably the solvent is selected from the group consisting of methyl *tert*-butyl ether, cyclohexane, toluene and acetone, wherein most preferably the solvent is acetone.

**6.** The process as claimed in one of claims 1 to 4, wherein the reaction mixture is free of any additional solvent.

**7.** The process as claimed in one of the preceding claims, wherein the esterase, optionally immobilized, is suspended in the reaction mixture, wherein preferably the remaining reaction mixture is a solution.

**8.** The process as claimed in one of the preceding claims, wherein the compounds **I** and **II** are reacted with one another at a temperature of at least 10 °C, preferably at least 25 °C, more preferably at least 35 °C, most preferably at least 38 °C, and/or wherein the compounds **I** and **II** are reacted with one another at a temperature of 100 °C or less, preferably 60 °C or less, more preferably 45 °C or less, most preferably 42 °C or less.

**9.** The process as claimed in one of the preceding claims, wherein, in the reaction mixture after reacting compounds **I** and **II** are reacted with one another, the compound **III** has a final concentration denoted by $c_{III}^f$ and compound **IV** represented by the following formula

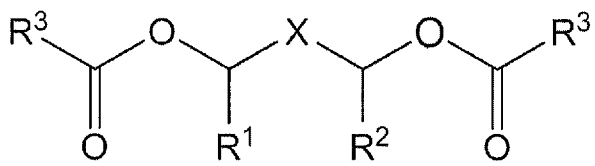

**(IV)**

being formed as a by-product when reacting compounds **I** and **II** is denoted by $c_{IV}^f$, wherein -X-, -$R^1$, -$R^2$, -$R^3$, and -$R^4$ are defined as in claim 1, wherein the ratio of $c_{III}^f$ and $c_{IV}^f$, $c_{III}^f / c_{IV}^f$, is at least 2, preferably at least 4, more preferably at least 10, most preferably at least 20.

**10.** The process as claimed in one of the preceding claims, wherein, in the reaction mixture after reacting compounds **I** and **II** are reacted with one another, the compound **III** has a final concentration denoted by $c_{III}^f$, wherein the ratio of $c_{III}^f$ and $c_I^0$, $c_{III}^f / c_I^0$, is at least 0.2, preferably at least 0.5, more preferably at least 0.75, most preferably at least 0.9.

**11.** The process as claimed in any of the preceding claims, wherein -$R^1$ and -$R^2$ are independently selected from the group consisting of hydrogen and aliphatic chemical groups; wherein preferably -$R^1$ and -$R^2$ are independently selected from the group consisting of hydrogen and unbranched or branched alkyl; wherein more preferably -$R^1$ and -$R^2$ are independently selected from the group consisting of hydrogen, methyl, ethyl, n-propyl, isopropyl, n-butyl, isobutyl, sec-butyl, and *tert*-butyl; wherein most preferably -$R^1$ and -$R^2$ both are hydrogen.

**12.** The process as claimed in any of the preceding claims, wherein -$R^3$ and -$R^4$ are independently selected from the group consisting of hydrogen and aliphatic chemical groups; wherein preferably -$R^3$ and -$R^4$ are independently selected from the group consisting of hydrogen and unbranched or branched alkyl; wherein more preferably -$R^3$ is selected from the group consisting of hydrogen, methyl, ethyl, *n*-propyl, isopropyl, *n*-butyl, isobutyl, *sec*-butyl, *tert*-butyl and/or -$R^4$ is selected from the group consisting of hydrogen, methyl, ethyl, *n*-propyl, isopropyl, vinyl, propenyl, allyl, isopropenyl; wherein most preferably -$R^3$ is methyl and/or -$R^4$ is methyl or ethyl.

Europäisches Patentamt
European Patent Office
Office européen des brevets

# EUROPEAN SEARCH REPORT

Application Number
EP 18 15 2653

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TOMER ET AL: "Enzymatic monoesterification of symmetric diols: restriction of molecular conformations influences selectivity", ORGANIC & BIOMOLECULAR CHEMISTRY, vol. 15, 2017, pages 8990-8997, XP002783348, * See pages 8990-8991 (Introduction), pages 8992-8993 (Tables 1-2), and page 8994 (left column) * ----- | 1-15 | INV. C12P7/04 C12P7/24 C12P7/62 |
| A | PAWAR ET AL: "Kinetics and mechanism of regioselective monoacetylation of 3-aryloxy-1,2-propandiols using immobilized Candida antarctica lipase", JOURNAL OF INDUSTRIAL AND ENGINEERING CHEMISTRY, vol. 31, 2015, pages 335-342, XP002783385, * See pages 338-339 (Effect of concentration...) * ----- | 1-15 | |
| | | | TECHNICAL FIELDS SEARCHED (IPC) |
| A | HAMBERG ET AL: "Selective monoacylation of diols by substrate assisted catalysis in T40A Candida antarctica Lipase B", CHEMCATCHEM, vol. 5, 2013, pages 743-747, XP002783349, * See pages 746-747 (Experimental section) * ----- | 1-15 | C12P |
| A | FRAMIS ET AL: "Lipase-catalysed selective monoacylation of 1,n-diols with vinyl acetate", TETRAHEDRON LETTERS, vol. 45, 2004, pages 5031-5033, XP004512224, * See pages 5032-5033 (bridging paragraph) * ----- -/-- | 1-15 | |

The present search report has been drawn up for all claims

3

| Place of search | Date of completion of the search | Examiner |
|---|---|---|
| Berlin | 26 July 2018 | Korsner, Sven-Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

## EUROPEAN SEARCH REPORT

Application Number
EP 18 15 2653

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| A | GILL ET AL: "Enantioselectivity of lipase-catalysed transesterification of 2-ethyl-1,3-propanediol: comparison of lipases from bacterial, fungal and animal sources", TETRAHEDRON : ASYMMETRY, vol. 8, 1997, pages 2227-2230, XP004083218, * See page 2228 (Table 1) and page 2229 (concentrations) * ----- | 1-15 | |
| A | HSIAO ET AL: "Selective monoacetylation of diol compounds by Aspergillus niger lipase", BIOTECHNOLOGY LETTERS, vol. 18, 1996, pages 1277-1282, XP002783351, * See page 1279 * ----- | 1-15 | |
| | | | TECHNICAL FIELDS SEARCHED (IPC) |

The present search report has been drawn up for all claims

| Place of search | Date of completion of the search | Examiner |
|---|---|---|
| Berlin | 26 July 2018 | Korsner, Sven-Erik |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011033039 A1


### Non-patent literature cited in the description


- **A. HAMBERG et al.** *ChemCatChem,* 2013, vol. 5, 743-747
- **V. FRAMIS et al.** *Tetrahedron Letters,* 2004, vol. 45, 5031-5033